(19) Europäisches Patentamt / European Patent Office / Office européen des brevets

(11) **EP 1 530 601 B1**

(12) **EUROPÄISCHE PATENTSCHRIFT**

(45) Veröffentlichungstag und Bekanntmachung des Hinweises auf die Patenterteilung:
**19.02.2020 Patentblatt 2020/08**

(21) Anmeldenummer: **03793635.8**

(22) Anmeldetag: **24.07.2003**

(51) Int Cl.:
***A61K 8/81*** *(2006.01)* ***C08F 226/06*** *(2006.01)*
***C08F 226/10*** *(2006.01)* ***C08F 226/02*** *(2006.01)*
***C08F 2/38*** *(2006.01)* ***A61Q 5/02*** *(2006.01)*
***A61Q 5/12*** *(2006.01)* ***A61Q 19/10*** *(2006.01)*

(86) Internationale Anmeldenummer:
**PCT/EP2003/008097**

(87) Internationale Veröffentlichungsnummer:
**WO 2004/022616 (18.03.2004 Gazette 2004/12)**

(54) **VERWENDUNG VON VERNETZTEN KATIONISCHEN COPOLYMEREN MIT REGLERN IN HAARKOSMETISCHEN ZUBEREITUNGEN UND ALS KONDITIONIERUNGSMITTEL IN KOSMETISCHEN ZUBEREITUNGEN**

USE OF CROSS-LINKED CATIONIC COPOLYMERS COMPRISING REGULATORS IN COSMETIC PREPARATIONS FOR HAIR AND AS A CONDITIONING AGENT IN COSMETIC PREPARATIONS

UTILISATION DE COPOLYMÈRES CATIONIQUES RÉTICULÉS COMPRENANT DES RÉGULATEURS DANS DES PRÉPARATIONS COSMÉTIQUES CAPILLAIRES ET COMME AGENT DE CONDITIONNEMENT DANS DES PRÉPARATIONS COSMÉTIQUES

(84) Benannte Vertragsstaaten:
**AT BE BG CH CY CZ DE DK EE ES FI FR GB GR HU IE IT LI LU MC NL PT RO SE SI SK TR**

(30) Priorität: **12.08.2002 DE 10237378**

(43) Veröffentlichungstag der Anmeldung:
**18.05.2005 Patentblatt 2005/20**

(73) Patentinhaber: **BASF SE**
**67056 Ludwigshafen am Rhein (DE)**

(72) Erfinder:
- **CHRISSTOFFELS, Lysander**
  **67117 Limburgerhof (DE)**
- **ANGEL, Maximilian**
  **67105 Schifferstadt (DE)**
- **HÖSSEL, Peter**
  **67105 Schifferstadt (DE)**
- **MATHAUER, Klemens**
  **69115 Heidelberg (DE)**
- **WOOD, Claudia**
  **69469 Weinheim (DE)**
- **FAUL, Dieter**
  **67150 Niederkirchen (DE)**

(74) Vertreter: **BASF IP Association**
**BASF SE**
**G-FLP-C6**
**67056 Ludwigshafen (DE)**

(56) Entgegenhaltungen:
**EP-A- 0 574 335** **EP-A- 0 893 117**
**WO-A-00/05274** **WO-A-96/37525**

- **ANONYMOUS: "Dispersants and hyperdispersants and their applications" RESEARCH DISCLOSURE, KENNETH MASON PUBLICATIONS, HAMPSHIRE, GB, Bd. 443, Nr. 109, März 2001 (2001-03), XP007127825 ISSN: 0374-4353**
- **ANONYMOUS: "Cationic polymeric thickeners useful in fabric softeners" RESEARCH DISCLOSURE, KENNETH MASON PUBLICATIONS, HAMPSHIRE, GB, Bd. 429, Nr. 116, Januar 2000 (2000-01), XP007125401 ISSN: 0374-4353**
- **PATENT ABSTRACTS OF JAPAN vol. 1997, no. 05, 30. Mai 1997 (1997-05-30) -& JP 09 003793 A (SUMITOMO CHEM CO LTD), 7. Januar 1997 (1997-01-07)**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 530 601 B1

## Beschreibung

Die vorliegende Erfindung betrifft die Verwendung von Polymeren, erhältlich durch radikalisch initiierte Copolymerisation von Monomergemischen aus

(a) 2 bis 70 Gew.-% eines kationischen Monomeren ausgewählt aus der Gruppe bestehend aus 3-Methyl-1-vinylimidazoliumchlorid und -methosulfat,
(b) 22 bis 97,98 Gew.-% N-Vinylpyrrolidon,
(c) 0 bis 40 Gew.-% eines weiteren radikalisch copolymerisierbaren Monomeren,
(d) 0,01 bis 8 Gew.-% mindestens einem als Vernetzer wirkenden Monomeren mit mindestens zwei ethylenisch ungesättigten, nichtkonjugierten Doppelbindungen und
(e) 0,01 bis 8 Gew.-% eines Reglers in haarkosmetischen Zubereitungen sowie die Verwendung dieser Polymere als Konditioniermittel in kosmetischen Zubereitungen.

Kationische Polymere werden als Konditioniermittel in kosmetischen Formulierungen eingesetzt. Anforderungen an Haarkonditioniermittel sind z.B. eine starke Reduktion der erforderlichen Kämmkraft im nassen wie auch im trockenen Haar, gute Entwirrung beim ersten Durchkämmen (engl. "Detangling") und gute Verträglichkeit mit weiteren Formulierungskomponenten. Außerdem verhindern kationische Polymere die elektrostatische Aufladung des Haares.

In Shampoos werden vor allem kationische Zellulose-Derivate (Polyquaternium-10) oder Guar-Gum Derivate eingesetzt. Allerdings beobachtet man bei diesen Verbindungen einen build-up Effekt, d.h. das Haar wird bei mehrfacher Anwendung mit dem Conditioner belegt und fühlt sich beschwert an.

Für die Konditionierung und Festigung von keratinösen Substanzen wie Haar, Nägel und Haut werden seit Jahren auch synthetische Polymere eingesetzt. Zudem werden synthetische Polymere in kosmetischen Formulierungen, die Pigmente oder kosmetisch wirksame Aktivkomponenten enthalten, als Verträglichkeitsvermittler zur Erreichung einer homogenen, stabilen Formulierung eingesetzt.

Zum Beispiel finden Copolymere aus Acrylamid und Dimethyldiallylammoniumchlorid (Polyquaternium 7) Verwendung. Diese haben allerdings den Nachteil hoher Restmonomerengehalte, da Acrylamid und Dimethyldiallylammoniumchlorid ungünstige Copolymerisations-parameter aufweisen.

Trotz der umfangreichen Bemühungen besteht nach wie vor Verbesserungsbedarf bei Polymeren zur Erzeugung elastischer Frisuren bei gleichzeitig starker Festigung auch bei hoher Luftfeuchtigkeit, guter Auswaschbarkeit und gutem Griff des Haares. Der Verbesserungsbedarf besteht ebenso bei Polymeren zur Erzeugung von gut kämmbarem, entwirrbarem Haar und zur Konditionierung von Haut und Haar in ihren sensorisch erfassbaren Eigenschaften wie Griff, Volumen, Handhabbarkeit usw. Ferner sind klare wässrige Zubereitungen dieser Polymere wünschenswert, die sich demnach durch eine gute Verträglichkeit mit anderen Formulierungsbestandteilen auszeichnen.

Weiterhin besteht Bedarf nach Polymeren, die als Konditioniermittel für kosmetische Zubereitungen geeignet sind und die mit einem hohen Feststoffgehalt hergestellt werden können. Von besonderem Interesse sind Polymere, die einen hohen Feststoffgehalt haben, eine geringe Viskosität aufweisen unter gleichzeitigen guten anwendungstechnischen Eigenschaften (wie beispielsweise Kämmbarkeit).

Aufgabe der vorliegenden Erfindung war es, ein kationisches Konditioniermittel für kosmetische Zubereitungen, insbesondere Shampoos zu finden, welche die genannten Nachteile nicht aufweist.

Quaternisierte Polymere und ihre Verwendung als Konditioniermittel in Haarpflegeformulierungen sind bekannt.

Kationische Polymere werden häufig als Konditioniermittel in haarkosmetischen Formulierungen eingesetzt. Sie bewirken in erster Linie eine Verbesserung der Nasskämmbarkeit des Haares. Außerdem verhindern kationische Polymere die elektrostatische Aufladung des Haares.

So wird z.B. in der EP-A-0 246 580 die Verwendung von unvernetzten Homo- und Copolymeren von 3-Methyl-1-vinylimidazolium-chloriden in kosmetischen Mitteln beschrieben. Die EP-A-0 544 158 und US-A-4,859,756 beanspruchen die Verwendung von unvernetzten Homo- und Copolymeren von chloridfreien, quaternisierten N-Vinylimidazolen in kosmetischen Zubereitungen. Aus der EP-A-0 715 843 ist die Verwendung von unvernetzten Copolymeren aus einem quaternisierten N-Vinylimidazol, N-Vinylcaprolactam und N-Vinylpyrrolidon sowie optional einem weiteren Comonomer in kosmetischen Zubereitungen bekannt.

Die DE-A-28 21 239 (US-A-4,348,380) und DE-A-31 06 974 beschreiben Copolymere von quaternisierten Diallylammoniumverbindungen in haarkosmetischen Zubereitungen.
Die US-A-5,275,809, EP-A-0 522 755, EP-A-0 521 665 und EP-A-0 521 666 offenbaren Copolymere mit Dimethyldiallylammoniumchlorid für die Verwendung in Shampoos. In keiner der vorstehend genannten Schriften ist ein vernetztes Polymer beschrieben.

Weiterhin werden auch vernetzten kationischen Copolymere und deren Verwendung als wasserlösliche und wasserunlösliche Zusätze in verschiedenste Bereichen beschrieben.
Die US-A-4,806,345 beschreibt vernetzte kationische Verdicker für kosmetische Formulierungen aus quaterniertem

Dimethylaminoethylmethacrylat und Acrylamid.

Die WO 93/25595 beschreibt vernetzte kationische Copolymere auf Basis quaternisierter Dialkylaminoalkylacrylate oder Dialkylaminoalkylacrylamiden. Als Anwendung wird der Einsatz dieser vernetzten Copolymere als Verdicker in kosmetischen Zubereitungen vorgeschlagen. Diese Polymere enthalten keine Regler.

DE 3 209 224 beschreibt die Herstellung von vernetzten Polymerisaten auf Basis N-Vinylpyrrolidon und (quaternisiertem) N-Vinylimidazol. Diese Polymerisate werden für die Verwendung als Adsorbentien und Ionenaustauscher beansprucht. Sie sind hochvernetzt, wasserunlöslich, wenig quellbar und daher nicht geeignet als Konditioniermittel in kosmetischen Formulierungen.

Vernetzte, agglomerierte Vinylimidazol-Copolymerisate werden in der WO 96/26229 als Farbstoffübertragungsinhibitoren genannt. Sie sind hochvernetzt, wasserunlöslich, wenig quellbar und daher nicht geeignet als Konditioniermittel in kosmetischen Formulierungen.

Aus der US-A-4,058,491 sind vernetzte kationische Hydrogele aus N-Vinylimidazol oder N-Vinylpyrrolidon und einem quaternisierten basischen Acrylat sowie weiteren Comonomeren bekannt. Diese Gele werden zur Komplexierung und kontrollierten Freisetzung anionischer Wirksubstanzen vorgeschlagen.

Die DE-A-42 13 971 beschreibt Copolymerisate aus einer ungesättigten Carbonsäure, quaternisiertem Vinylimidazol und optional weiteren Monomeren und einem Vernetzer. Die Polymere werden als Verdickungs- und Dispergiermittel vorgeschlagen. Die Verfahrensweise des Verdickens durch Protonierung eines wasserlöslichen, vernetzten Aminoalkyl(meth)acrylat wird in der EP-A-0 624 617 und EP-A-0 027 850 beschrieben.

Die WO 96/37525 beschreibt die Herstellung von vernetzten Copolymeren aus u.a. N-Vinylpyrrolidon und quaternisierten Vinylimidazolen in Gegenwart von Polymerisationsreglern und ihre Verwendung insbesondere in Waschmitteln.

Die WO 97/35544 beschreibt die Verwendung von vernetzten kationischen Polymeren mit Dialkylaminoalkyl(meth)acrylaten bzw. -(meth)acryl-amiden in Shampoozusammensetzungen.

Die DE-A-197 31 907 beschreibt die Verwendung von vernetzten kationischen Copolymeren, die N-Vinylimidazole enthalten, in haarkosmetischen Formulierungen.

EP 0 893 117 und EP 1 064 924 beschreiben hochmolekulare vernetzte Polymere, die eine gute konditionierende Wirkung in Shampoos aufweisen, während die entsprechenden niedermolekularen unvernetzten Polymere nur eine geringe Wirksamkeit als Konditioniermittel zeigen.

Nachteil dieser obengenannten Erfindungen ist, dass die Herstellung dieser Polymerisate als Lösungen bei sehr niedriger Feststoffgehalt erfolgt weil anders die Viskositäten dieser Lösungen zu hoch sind. Zusätzlicher Nachteil ist die Erzeugung eines relativ großen Anteil von ungelösten Gelpartikeln. Dies führt zu einer Vielzahl von anwendungstechnischen Nachteilen, wie beispielsweise längere Polymerisationszeiten, lange Filtrations- und Abfüllzeiten. Aufgrund des geringen Feststoffgehaltes ergeben sich hohe Kosten bei der Herstellung (Kesselkapazitäten) sowie hohe Transportkosten.

Aufgabe der vorliegenden Erfindung war es Polymerisate zur Verfügung zu stellen, die sich für kosmetischen Zubereitungen, insbesondere haarkosmetische Zubereitungen eignen (konditionierende Eigenschaften), eine befriedigenden Viskosität besitzen und dabei gleichzeitig mit einem hohen Feststoffgehalt herstellbar sind. Von besonderem Interesse waren Polymerisate, welche weniger Gelpartikel als die Polymerisate des Standes der Technik, insbesondere der EP 0 893 117, enthalten.

Es wurde gefunden, dass die erfindungsgemäßen verwendeten Polymerisate diese Aufgabe lösen.

Die erfindungsgemäße Verwendung betrifft Polymere, die erhältlich sind durch

(i) radikalisch initiierte Copolymerisation von Monomergemischen aus

(a) 2 bis 70 Gew.-% eines kationischen Monomeren ausgewählt aus der Gruppe bestehend aus 3-Methyl-1-vinyl-imidazoliumchlorid und methosulfat
(b) 22 bis 97,98 Gew.-% N-Vinylpyrrolidon,
(c) 0 bis 40 Gew.-% eines weiteren radikalisch copolymerisierbaren Monomeren,
(d) 0,01 bis 8 Gew.-% mindestens einem als Vernetzer wirkenden Monomeren mit mindestens zwei ethylenisch ungesättigten, nichtkonjugierten Doppelbindungen und
(e) 0,01 bis 8 Gew.-% eines Reglers.

Die so erhältlichen Polymere eignen sich für die Verwendung in haarkosmetischen Zubereitungen.

Die so erhältlichen Polymere eignen sich als Konditioniermittel in kosmetischen Zubereitungen, insbesondere in haut- und/oder haarkosmetischen Zubereitungen.

Bevorzugt ist die Verwendung in Shampoos.

Die Monomere (a) sind ausgewählt aus der Gruppe bestehend aus 3-Methyl-1-vinyl-imidazoliumchlorid und -methosulfat.

Das Monomer der Gruppe (b) ist N-Vinylpyrrolidon.

Als von Monomeren (a) und (b) verschiedene Monomere (c) eignen sich $C_1$-$C_{40}$-Alkylester der (Meth) acrylsäure, wobei die Ester abgeleitet werden von linearen, verzweigtkettigen oder carbocyclischen Alkoholen, z.B. Methyl(meth)acrylat, Ethyl(meth)-acrylat, tert.-Butyl(meth)acrylat, Isobutyl (meth)acrylat, n-Butyl(meth)acrylat, Stearyl(meth)acrylat, oder Ester von alkoxylierten Fettalkoholen, z.B. $C_1$-$C_{40}$-Fettalkoholen, umgesetzt mit Ethylenoxid, Propylenoxid oder Butylenoxid, insbesondere $C_{10}$-$C_{18}$-Fettalkohole, umgesetzt mit 3 bis 150 Ethylenoxid-einheiten. Weiterhin eignen sich N-Alkyl-substituierte Acrylamide mit linearen, verzweigtkettigen oder carbocyclischen Alkylresten wie N-tert.-Butylacrylamid, N-Butylacrylamid, N-Octylacrylamid, N-tert.-Octylacrylamid.

Ferner eignen sich Styrol, Vinyl- und Allylester von $C_1$-$C_{40}$-Carbonsäuren, die linear, verzweigtkettig oder carbocyclisch sein können, z.B. Vinylacetat, Vinylpropionat, Vinylneononanoat, Vinylneoundekansäure, t-Butyl-benzoesäurevinylester, Alkylvinylether, beispielsweise Methylvinylether, Ethylvinylether, Butylvinylether, Stearylvinylether.

Acrylamide, wie N-tert.-Butylacrylamid, N-Butylacrylamid, N-Octylacrylamid, N-tert.-Octylacrylamid und N-Alkyl-substituierte Acrylamide mit linearen, verzweigtkettigen oder carbocyclischen Alkylresten, wobei der Alkylrest die oben für $R^4$ angegebenen Bedeutungen besitzen kann.

Als Monomere (c) eignen sich insbesondere $C_1$ bis $C_{24}$-, ganz besonders $C_1$ bis $C_{10}$-Alkylester der (Meth)acrylsäure, z.B. Methyl(meth)acrylat, Ethyl(meth)acrylat, tert.-Butyl(meth)-acrylat, Isobutyl(meth)acrylat, n-Butyl(meth)acrylat und Acrylamide wie N-tert.-Butylacrylamid oder N-tert.-Octylacrylamid. Falls die Polymere Monomere der Gruppe (c) enthalten, so können sie in Mengen bis zu 40 Gew.-%, bevorzugt bis zu 30 Gew.-% darin enthalten sein.

Monomere (d), die eine vernetzende Funktion besitzen, sind Verbindungen mit mindestens 2 ethylenisch ungesättigten, nichtkonjugierten Doppelbindungen im Molekül.

Geeignete Vernetzer (d) sind zum Beispiel Acrylester, Methacrylester, Allylether oder Vinylether von mindestens zweiwertigen Alkoholen. Die OH-Gruppen der zugrundeliegenden Alkohole können dabei ganz oder teilweise verethert oder verestert sein; die Vernetzer enthalten aber mindestens zwei ethylenisch ungesättigte Gruppen.

Beispiele für die zugrundeliegenden Alkohole sind zweiwertige Alkohole wie 1,2-Ethandiol, 1,2-Propandiol, 1,3-Propandiol, 1,2-Butandiol, 1,3-Butandiol, 2,3-Butandiol, 1,4-Butandiol, But-2-en-1,4-diol, 1,2-Pentandiol, 1,5-Pentandiol, 1,2-Hexandiol, 1,6-Hexandiol, 1,10-Decandiol, 1,2-Dodecandiol, 1,12-Dodecandiol, Neopentylglykol, 3-Methylpentan-1,5-diol, 2,5-Dimethyl-1,3-hexandiol, 2,2,4-Trimethyl--1,3-pentandiol, 1,2-Cyclohexandiol, 1,4-Cyclohexandiol, 1,4-Bis(hydroxymethyl)cyclohexan, Hydroxypivalinsäure-neopentylglykolmonoester, 2,2-Bis(4-hydroxyphenyl)-propan, 2,2-Bis[4-(2-hydroxypropyl)phenyl]propan, Diethylenglykol, Triethylenglykol, Tetraethylenglykol, Dipropylenglykol, Tripropylenglykol, Tetrapropylenglykol, 3-Thio-pentan-1,5-diol, sowie Polyethylenglykole, Polypropylenglykole und Polytetrahydrofurane mit Molekulargewichten von jeweils 200 bis 10000. Außer den Homopolymerisaten des Ethylenoxids bzw. Propylenoxids können auch Blockcopolymerisate aus Ethylenoxid oder Propylenoxid oder Copolymerisate, die Ethylenoxid- und Propylenoxid-Gruppen eingebaut enthalten, eingesetzt werden. Beispiele für zugrundeliegende Alkohole mit mehr als zwei OH-Gruppen sind Trimethylolpropan, Glycerin, Pentaerythrit, 1,2,5-Pentantriol, 1,2,6-Hexantriol, Triethoxycyanursäure, Sorbitan, Zucker wie Saccharose, Glucose, Mannose. Selbstverständlich können die mehrwertigen Alkohole auch nach Umsetzung mit Ethylenoxid oder Propylenoxid als die entsprechenden Ethoxylate bzw. Propoxylate eingesetzt werden. Die mehrwertigen Alkohole können auch zunächst durch Umsetzung mit Epichlorhydrin in die entsprechenden Glycidylether überführt werden.

Weitere geeignete Vernetzer sind die Vinylester oder die Ester einwertiger, ungesättigter Alkohole mit ethylenisch ungesättigten $C_3$- bis $C_6$-Carbonsäuren, beispielsweise Acrylsäure, Methacrylsäure, Itaconsäure, Maleinsäure oder Fumarsäure. Beispiele für solche Alkohole sind Allylalkohol, 1-Buten-3-ol, 5-Hexen-1-ol, 1-Octen-3-ol, 9-Decen-1-ol, Dicyclopentenylalkohol, 10-Undecen-1-ol, Zimtalkohol, Citronellol, Crotylalkohol oder cis-9-Octadecen-1-ol. Man kann aber auch die einwertigen, ungesättigten Alkohole mit mehrwertigen Carbonsäuren verestern, beispielsweise Malonsäure, Weinsäure, Trimellitsäure, Phthalsäure, Terephthalsäure, Citronensäure oder Bernsteinsäure.

Weitere geeignete Vernetzer sind Ester ungesättigter Carbonsäuren mit den oben beschriebenen mehrwertigen Alkoholen, beispielsweise der Ölsäure, Crotonsäure, Zimtsäure oder 10-Undecensäure.

Geeignet als Monomere (d) sind außerdem geradkettige oder verzweigte, lineare oder cyclische, aliphatische oder aromatische Kohlenwasserstoffe, die über mindestens zwei Doppelbindungen verfügen, die bei aliphatischen Kohlenwasserstoffen nicht konjugiert sein dürfen, z.B. Divinylbenzol, Divinyltoluol, 1,7-Octadien, 1,9-Decadien, 4-Vinyl-1-cyclohexen, Trivinylcyclohexan oder Polybutadiene mit Molekulargewichten von 200 bis 20000.

Als Vernetzer sind ferner geeignet die Acrylsäureamide, Methacrylsäureamide und N-Allylamine von mindestens zweiwertigen Aminen. Solche Amine sind zum Beispiel 1,2-Diaminomethan, 1,2-Diaminoethan, 1,3-Diaminopropan, 1,4-Diaminobutan, 1,6-Diaminohexan, 1,12-Dodecandiamin, Piperazin, Diethylentriamin oder Isophorondiamin. Ebenfalls geeignet sind die Amide aus Allylamin und ungesättigten Carbonsäuren wie Acrylsäure, Methacrylsäure, Itaconsäure, Maleinsäure, oder mindestens zweiwertigen Carbonsäuren, wie sie oben beschrieben wurden.

Ferner sind Triallylamin und Triallylmonoalkylammoniumsalze, z.B. Triallylmethylammoniumchlorid oder -methylsulfat, als Vernetzer geeignet.

Geeignet sind auch N-Vinyl-Verbindungen von Harnstoffderivaten, mindestens zweiwertigen Amiden, Cyanuraten oder Urethanen, beispielsweise von Harnstoff, Ethylenharnstoff, Propylenharnstoff oder Weinsäurediamid, z.B. N,N'-Divinylethylenharnstoff oder N,N'-Divinylpropylenharnstoff.

Weitere geeignete Vernetzer sind Divinyldioxan, Tetraallylsilan oder Tetravinylsilan.

Selbstverständlich können auch Mischungen der vorgenannten Verbindungen eingesetzt werden. Vorzugsweise werden solche Vernetzer eingesetzt, die in der Monomermischung löslich sind.

Besonders bevorzugt eingesetzte Vernetzer sind beispielsweise Methylenbisacrylamid, Triallylamin und Triallylalkylammoniumsalze, Divinylimidazol, Pentaerythrittriallylether, N,N'-Divinylethylenharnstoff, Umsetzungsprodukte mehrwertiger Alkohole mit Acrylsäure oder Methacrylsäure, Methacrylsäureester und Acrylsäureester von Polyalkylenoxiden oder mehrwertigen Alkoholen, die mit Ethylenoxid und/oder Propylenoxid und/oder Epichlorhydrin umgesetzt worden sind.

Ganz besonders bevorzugt als Vernetzer sind Pentaerythrittriallylether, Methylenbisacrylamid, N,N'-Divinylethylenharnstoff, Triallylamin und Triallylmonoalkylammoniumsalze, und Acrylsäureester von Glykol, Butandiol, Trimethylolpropan oder Glycerin oder Acrylsäureester von mit Ethylenoxid und/oder Epichlorhydrin umgesetzten Glykol, Butandiol, Trimethylolpropan oder Glycerin.

Die Monomere (a) bis (e) können jeweils einzeln oder im Gemisch mit anderen Monomeren der gleichen Gruppe eingesetzt werden.

Die Copolymerisation erfolgt in Gegenwart mindestens eines Reglers (e). Als Regler (Polymerisationsregler) werden Verbindungen mit hohen Übertragungskonstanten bezeichnet. Regler beschleunigen Kettenübertragungsreaktionen und bewirken damit eine Herabsetzung des Polymerisationsgrades der resultierenden Polymeren, ohne die Bruttoreaktions-Geschwindigkeit zu beeinflussen.

Bei den Reglern kann man zwischen mono-, bi- oder polyfunktionalen Regler unterscheiden je nach Anzahl der funktionellen Gruppen im Molekül, die zu einen oder mehreren Kettenübertragungsreaktionen führen können. Geeignete Regler werden beispielsweise ausführlich beschrieben von K.C. Berger und G. Brandrup in J. Brandrup, E.H. Immergut, Polymer Handbook, 3. Aufl., John Wiley & Sons, New York, 1989, S. II/81 - II/141. Als Regler eignen sich beispielsweise Aldehyde wie Formaldehyd, Acetaldehyd, Propionaldehyd, n-Butyraldehyd, Isobutyraldehyd. Ferner können auch als Regler eingesetzt werden: Ameisensäure, ihre Salze oder Ester, 2,5-Diphenyl-1-hexen, Ammoniumformiat, Hydroxylammoniumsulfat, und Hydroxylammoniumphosphat.

Weitere geeignete Regler sind Halogenverbindungen wie Alkylhalogenide, wie Tetrachlormethan, Chloroform, Bromtrichlormethan, Bromoform, Allylbromid, und Benzylverbindungen, wie Benzylchlorid oder Benzylbromid.

Weitere geeignete Regler sind Allylverbindungen, wie z.B. Allylalkohol, funktionale Allylether, wie Allyl Ethoxylate, alkyl allyl ether, oder Glycerin Monoallylether.

Bevorzugt werden als Regler Verbindungen eingesetzt, die Schwefel in gebundener Form enthalten.

Verbindungen dieser Art sind beispielsweise anorganische Hydrogensulfite, Disulfite und Dithionite oder organische Sulfide, Disulfide, Polysulfide, Sulfoxide, Sulfone. Folgende Regler werden beispielhaft genannt: Di-n-butylsulfid, Di-n-octylsulfid, Diphenylsulfid, Thiodiglykol, Ethylthioethanol, Diisopropyldisulfid, Di-n-butyldisulfid, Di-n-hexyldisulfid, Diacetyldisulfid, Diethanolsulfid, Di-t-butyltrisulfid, Dimethylsulfoxid, Dialkylsulfid, Dialkyldisulfid und/oder Diarylsulfid.

Besonders bevorzugt sind organische Verbindungen, die Schwefel in gebundener Form enthalten.

Bevorzugt als Polymerisationsregler eingesetzte Verbindungen sind Thiole (Verbindungen, die Schwefel in Form von SH-Gruppen erhalten, auch als Mercaptane bezeichnet). Bevorzugt sind als Regler mono-, bi- und polyfunktionale Mercaptane, Mercaptoalkohole und/oder Mercaptocarbonsäuren.

Beispiele für diese Verbindungen sind Allylthioglykolate, Ethylthioglykolat, Cystein, 2-Mercaptoethanol, 1,3-Mercaptopropanol, 3-Mercaptopropan-1,2-diol, 1,4-Mercaptobutanol, Mercaptoessigsäure, 3-Mercaptopropionsäure, Mercaptobernsteinsäure, Thioglycerin, Thioessigsäure, Thioharnstoff und Alkylmercaptane wie n-Butylmercaptan, n-Hexylmercaptan oder n-Dodecylmercaptan.

Besonders bevorzugte Thiole sind Cystein, 2-Mercaptoethanol, 1,3-Mercaptopropanol, 3-Mercaptopropan-1,2-diol, Thioglycerin, Thioharnstoff.

Beispiele für bifunktionale Regler, die zwei Schwefel in gebundener Form enthalten sind bifunktionale Thiole wie z.B. Dimercaptopropansulfonsäure (Natrium Salz), Dimercaptobernsteinsäure, Dimercapto-1-propanol, Dimercaptoethan, Dimercaptopropan, Dimercaptobutan, Dimercaptopentan, Dimercaptohexan, Ethylenglykol-bis-thioglykolate und Butandiol-bis-thioglykolat.

Beispiele für polyfunktionale Regler sind Verbindungen, die mehr als zwei Schwefel in gebundener Form enthalten. Beispiele hierfür sind trifunktionale und/oder tetrafunktionale Mercaptane.

Bevorzugte trifunktionale Regler sind trifunktionale Mercaptane, wie z.B. Trimethylolpropan tris(2-mercaptoethanat), Trimethylolpropan tris(3-mercaptopropionat), Trimethylolpropan tris(4-mercaptobutanat), Trimethylolpropan tris(5-mercaptopentanat), Trimethylolpropan tris(6-mercaptohexanat), Trimethylolpropan tris(2-mercaptoacetat).

Glyceryl thioglycolat, glyceryl thiopropionat, glyceryl thioethylat, glycerylthiobutanat.

1,1,1-Propanetriyl tris-(Mercaptoacetat), 1,1,1-Propanetriyl tris-(Mercaptoethanat), 1,1,1-Propanetriyl tris-(Mercaptoproprionat), 1,1,1-Propanetriyl tris-(Mercaptobutanat) 2-hydroxymethyl-2-methyl-1,3-propandiol tris-(Mercaptoacetat),
2-hydroxymethyl-2-methyl-1,3-propandiol tris-(Mercaptoethanat),
2-hydroxymethyl-2-methyl-1,3-propandiol tris-(Mercaptopropionat),
2-hydroxymethyl-2-methyl-1,3-propandiol tris-(Mercaptobutanat).

Besonders bevorzugte trifunktionale Regler sind Glyceryl thioglycolat, Trimethylolpropan tris(2-mercaptoacetat, 2-hydroxymethyl-2-methyl-1,3-propanediol tris-(Mercaptoacetat). Bevorzugte tetrafunktionale Mercaptane sind Pentaerythritol tetraquis (2-mercaptoacetat) Pentaerythritol tetraquis (2-mercaptoethanat), Pentaerythritol tetraquis(3-mercaptopropionat), Pentaerythritol tetraquis-(4-mercaptobutanat), Pentaerythritol tetraquis(5-mercaptopentanat), Pentaerythritol tetraquis(6-mercaptohexanat).

Als weitere polyfunktionale Regler eignen sich Si-Verbindungen, die durch Umsetzung von Verbindungen der Formel (IVa) entstehen. Weiterhin eignen sich als polyfunktionale Regler Si-Verbindungen der Formel (IVb).

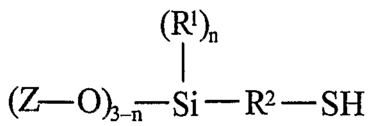
(IVa)

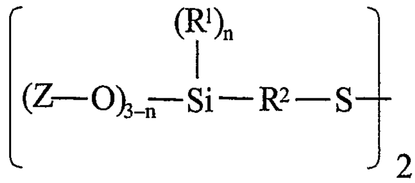
(IVb)

in der

- n ein Wert von 0 bis 2 ist,
- $R^1$ eine $C_1$-$C_{16}$-Alkylgruppe oder Phenylgruppe bedeutet
- $R^2$ eine $C_1$-$C_{18}$-Alkylgruppe, die Cyclohexyl-oder Phenylgruppe bezeichnet,
- Z für eine $C_1$-$C_{18}$ Alkylgruppe, $C_2$-$C_{18}$-Alkylengruppe oder $C_2$-$C_{18}$-Alkinylgruppe steht, deren Kohlenstoffatome durch nichtbenachbarte Sauerstoff- oder Halogenatome ersetzt sein können, oder für eine der Gruppen

$N{=}C(R_3)_2$ oder 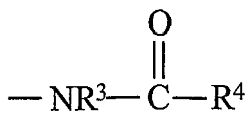

in denen

$R_3$ eine $C_1$-$C_{12}$-Alkylgruppe bedeutet und
$R_4$ eine $C_1$-$C_{18}$-Alkylgruppe bezeichnet.

Besonders bevorzugt sind die Verbindungen IVa, darunter vor allem Mercaptopropyltrimethoxysilan und Mercaptopropyltriethoxysilan.

Alle genannten Regler können einzeln oder in Kombination miteinander eingesetzt werden.

In einer bevorzugten Ausführungsform setzt man auf 1 Gew.-Teil eines Vernetzers (Monomer (d)) 0,1 bis 5, vorzugsweise 0,2 bis 2, und insbesondere 0,25 bis 1 Gew.-Teile eines Polymerisationsreglers (e) ein.

Die Herstellung der Polymerisate kann nach den an sich bekannten Verfahren der radikalisch initiierten Polymerisation, z.B. durch Lösungspolymerisation, Emulsionspolymerisation, Suspensionspolymerisation, Fällungspolymerisation, umgekehrte Suspensionspolymerisation oder umgekehrte Emulsionspolymerisation erfolgen, ohne dass die verwendbaren Methoden darauf beschränkt sind.

Die Monomeren (a), (d), (e) und gegebenenfalls (b) und (c) werden nach Art einer Lösungspolymerisation in Wasser und/oder polaren organischen Lösemitteln copolymerisiert. Geeignete polare organische Lösemittel sind beispielsweise mit Wasser mischbare Verbindungen wie Tetrahydrofuran, N-Methylpyrrolidon, Dioxan, Dimethylsulfoxid, Aceton, Glykole wie Ethylenglykol, Propylenglykol, Butandiol-1,4,diethylenglykol, Triethylenglykol, Tetraethylenglykol sowie Blockcopolymerisate aus Ethylenoxid und Propylenoxid sowie veretherte Polyalkylenglykole, die beispielsweise durch Alkylierung von Alkylenglykolen und Polyalkylenglykolen erhältlich sind. Geeignet sind beispielsweise die $C_1$- bis $C_4$-Alkylendgruppen enthaltenden Glykole oder Polyethylenglykole. Die Veretherung kann ein- oder auch beidseitig

erfolgen. Weitere geeignete Lösemittel sind Alkohole mit 1 bis 4 Kohlenstoffatomen oder Aceton. Man kann entweder ein einziges Lösemittel einsetzen oder die Copolymerisation auch in Gegenwart von Lösemittelmischungen durchführen. Besonders bevorzugte Lösemittel sind Wasser, $C_1$- bis $C_3$-Alkohole wie Methanol, Ethanol, Isopropanol und n-Propanol sowie Mischungen der genannten Lösemittel. Die Lösemittel werden üblicherweise in einer solchen Menge eingesetzt, dass man Copolymerisat-Lösungen mit einem Copolymerisat-Gehalt von 5 bis 80, vorzugsweise 10 bis 60 Gew.-% erhält.

In einer bevorzugten Ausführungsform wird der Regler (e), gegebenenfalls als Lösung in Wasser und/oder einem $C_1$-$C_4$ Alkohol dem Reaktionsansatz zudosiert.

In einer bevorzugten Ausführungsform werden die Verfahren als Batchfahrweise durchgeführt. Hierbei ist bevorzugt, den Regler (e) in der Vorlage vorzulegen.

In einer bevorzugten Ausführungsform werden die Verfahren als Zulauffahrweise durchgeführt. Dabei werden einzelne oder alle Reaktionsteilnehmer ganz oder teilweise, absatzweise oder kontinuierlich, gemeinsam oder in getrennten Zuläufen zu einer Reaktionsmischung gegeben. So kann man beispielsweise zu einem Gemisch der Monomeren und eines Lösemittels bei der Polymerisationstemperatur innerhalb einer gegebenen Zeit eine Lösung des Polymerisationsreglers und eine Initiatorlösung kontinuierlich oder absatzweise zugeben. Es ist jedoch auch möglich, eine Mischung aus Regler und Initiator der auf Polymerisationstemperatur erwärmten Vorlage zuzudosieren. Eine andere Variante besteht darin, den Initiator unterhalb oder bei der Polymerisationstemperatur in die Vorlage zu geben und nur den Regler oder eine Lösung des Reglers nach Erreichen der Polymerisationstemperatur innerhalb eines vorgegebenen Zeitraums dem Reaktionsgemisch zuzuführen. In einer weiteren Variante werden zu einem Gemisch aus Regler (e), Monomeren (a) und gegebenenfalls Monomeren (b) und (c) und einem Lösemittel der Initiator und der Vernetzer (d) nach Erreichen der Polymerisations-temperatur zugegeben. Man kann auch die Vorlage auf Polymerisationstemperatur erwärmen und dann Regler (e), Initiator und Monomere (d) in getrennten Zuläufen oder gemeinsam zugeben. Selbstverständlich können auch Regler (e), Initiator, Monomere (d) und Monomere (a) und gegebenenfalls Monomere (b) und (c) zu einer auf Polymerisationstemperatur erwärmten Vorlage gegeben werden. Vorzugsweise verwendet man Wasser oder ein Gemisch aus Wasser und mindestens einem Teil der Monomeren (a) und gegebenenfalls (b) und (c) sowie gegebenenfalls weitere Komponenten als Vorlage. Besonders bevorzugt ist hierbei eine Verfahrensweise, bei der die Polymerisationsregler (e) während der Polymerisation der Monomeren kontinuierlich oder portionsweise zudosiert werden.

Die Polymerisation erfolgt üblicherweise bei Temperaturen von 20°C bis 130°C und bei Normaldruck oder unter Eigendruck.

Als Initiatoren für die radikalische Polymerisation können die hierfür üblichen wasserlöslichen und wasserunlöslichen Peroxo- und/oder Azo-Verbindungen eingesetzt werden, beispielsweise Alkali- oder Ammoniumperoxidisulfate, Dibenzoylperoxid, tert.-Butylperpivalat, tert.-Butyl-per-2-ethylhexanoat, Di-tert.-butylperoxid, tert.-Butylhydroperoxid, Azo-bis-isobutyronitril, Azo-bis-(2-amidinopropan)dihydrochlorid oder 2,2'-Azo-bis-(2-methyl-butyronitril). Geeignet sind auch Initiatormischungen oder Redox-Initiator Systeme, wie z.B. Ascorbinsäure/Eisen(II)sulfat / Natriumperoxodisulfat, tert.-Butylhydroperoxid /Natriumdisulfit, tert.-Butylhydroperoxid/ Natriumhydroxymethansulfinat. Die Initiatoren können in den üblichen Mengen eingesetzt werden, beispielsweise 0,05 bis 5 Gew.-%, bezogen auf die Menge der zu polymerisierenden Monomeren.

Das Molekulargewicht und der K-Wert der Polymerisate lässt sich in an sich bekannter Weise durch die Wahl der Polymerisationsbedingungen, beispielsweise Polymerisationsdauer, Polymerisationstemperatur oder Initiatorkonzentration, und durch den Gehalt an Vernetzer, und Regler in einem breiten Bereich variieren.

In einer bevorzugten Ausführungsform wählt der Fachmann die Reaktionsbedingungen so, dass Polymerisate entstehen, welche eine Viskosität unter 15000 mPas (gemessen nach Brookfield, Spindel 4, 12 Upm, 25°C) aufweisen.

Die Konzentration der Monomeren im Reaktionsmedium beträgt üblicherweise 5 bis 60 und liegt vorzugsweise in dem Bereich von 10 bis 45 Gew.-%. Die Polymerisation wird so geführt, dass eine sichtbare Vergelung des Reaktionsansatzes unterbleibt. Sofern Gelteilchen entstehen sollten, haben diese einen Durchmesser von weniger als 1 mm, vorzugsweise weniger als 500 nm, bestimmt durch Streulichtmessung im gewählten Reaktionsmedium. Die entstehenden Copolymerisate sind in dem Reaktionsmedium homogen löslich.

Die K-Werte der Polymerisate liegen in einem Bereich zwischen 10 bis 350, vorzugsweise 20 bis 200 und besonders bevorzugt 35 bis 110. Die K-Werte werden nach Fikentscher, Cellulosechemie, Bd. 13, S. 58-64 (1932) bei 25°C 0,1 %ig in 5 Gew.-% Kochsalzlösung gemessen.

Die bei der Polymerisation entstandenen Mischungen können im Anschluss an den Polymerisationsprozess einer physikalischen oder chemischen Nachbehandlung unterworfen werden. Solche Verfahren sind beispielsweise die bekannten Verfahren zur Restmonomerenreduzierung wie z.B. die Nachbehandlung durch Zusatz von Polymerisationsinitiatoren oder Mischungen mehrerer Polymerisationsinitiatoren bei geeigneten Temperaturen oder Erhitzen der Polymerisationslösung auf Temperaturen oberhalb der Polymerisations-temperatur, eine Nachbehandlung der Polymerlösung mittels Wasserdampf oder Strippen mit Stickstoff oder Behandeln der Reaktionsmischung mit oxidierenden oder reduzierenden Reagenzien, Adsorptionsverfahren wie die Adsorption von Verunreinigung an ausgewählten Medien wie z.B. Aktivkohle oder eine Ultrafiltration. Es können sich auch die bekannten Aufarbeitungsschritte anschließen, beispielsweise geeignete Trockenverfahren wie Sprüh-, Gefrier- oder Walzentrocknung oder an die Trocknung anschließende Agglomerationsverfahren. Die nach dem erfindungsgemäßen Verfahren erhaltenen restmonomerenarmen Mischungen können auch direkt in den Handel gebracht werden.

Die Polymere werden vorteilhaft in kosmetischen Zubereitungen verwendet, insbesondere haarkosmetischen Zubereitungen.

Der Begriff der kosmetischen Zubereitungen ist breit zu verstehen und meint all solche Zubereitungen, die sich zum Auftragen auf Haut und/oder Haare und/oder Nägel eignen und einen anderen als einen ausschließlich medizinisch-therapeutischen Zweck verfolgen.

Die Polymere können in hautkosmetischen Zubereitungen eingesetzt werden.

Beispielsweise werden die erfindungsgemäßen Polymere in kosmetischen Mitteln zur Reinigung der Haut verwendet. Solche kosmetischen Reinigungsmittel sind ausgewählt aus Stückseifen, wie Toilettenseifen, Kernseifen, Transparentseifen, Luxusseifen, Deoseifen, Cremeseifen, Babyseifen, Hautschutzseifen, Abrasiveseifen und Syndets, flüssigen Seifen, wie pastöse Seifen, Schmierseifen und Waschpasten, und flüssigen Wasch-, Dusch- und Badepräparaten, wie Waschlotionen, Duschbädern und -gelen, Schaumbädern, Ölbädern und Scrub-Präparaten.

Bevorzugt werden die Polymere in kosmetischen Mitteln zur Pflege und zum Schutz der Haut, in Nagelpflegemitteln sowie in Zubereitungen für die dekorative Kosmetik angewendet.

Besonders bevorzugt ist die Verwendung in Hautpflegemitteln, Intimpflegemitteln, Fußpflegemitteln, Deodorantien, Lichtschutzmitteln, Repellents, Rasiermitteln, Haarentfernungsmitteln, Antiaknemitteln, Make-up, Maskara, Lippenstifte, Lidschatten, Kajalstiften, Eyelinern, Rouges, Pudern und Augenbrauenstiften.

Die Hautpflegemittel liegen insbesondere als W/O- oder O/W-Hautcremes, Tag- und Nachtcremes, Augencremes, Gesichtscremes, Antifaltencremes, Feuchthaltecremes, Bleichcremes, Vitamincremes, Hautlotionen, Pflegelotionen und Feuchthaltelotionen vor.

In den kosmetischen Zubereitungen können die Polymere besondere Wirkungen entfalten. Die Polymere können unter anderem zur Feuchthaltung und Konditionierung der Haut und zur Verbesserung des Hautgefühls beitragen. Die Polymere können auch als Verdicker in den Formulierungen wirken. Durch Zusatz der erfindungsgemäßen Polymere kann in bestimmten Formulierungen eine erhebliche Verbesserung der Hautverträglichkeit erreicht werden.

Die Copolymere sind in den hautkosmetischen Zubereitungen in einem Anteil von etwa 0,001 bis 20 Gew.-%, vorzugsweise 0,01 bis 10 Gew.-%, ganz besonders bevorzugt 0,1 bis 5 Gew.-%, bezogen auf das Gesamtgewicht des Mittels, enthalten.

Je nach Anwendungsgebiet können die Mittel in einer zur Hautpflege geeigneten Form, wie z.B. als Creme, Schaum, Gel, Stift, Pulver, Mousse, Milch oder Lotion appliziert werden.

Die hautkosmetischen Zubereitungen können neben den Polymeren und geeigneten Lösungsmitteln noch in der Kosmetik übliche Zusätze, wie Emulgatoren, Konservierungsmittel, Parfümöle, kosmetische Wirkstoffe wie Phytantriol, Vitamin A, E und C, Retinol, Bisabolol, Panthenol, Lichtschutzmittel, Bleichmittel, Färbemittel, Tönungsmittel, Bräunungsmittel (z.B. Dihydroxyaceton), Collagen, Eiweißhydrolysate, Stabilisatoren, pH-Wert-Regulatoren, Farbstoffe, Salze, Verdicker, Gelbildner, Konsistenzgeber, Silikone, Feuchthaltemittel, Rückfetter und weitere übliche Additive enthalten.

Als geeignete Lösungsmittel sind insbesondere zu nennen Wasser und niedrige Monoalkohole oder Polyole mit 1 bis 6 Kohlenstoffatomen oder Mischungen davon; bevorzugte Monoalkohole oder Polyole sind Ethanol, i-Propanol, Propylenglycol, Glycerin und Sorbit.

Als weitere übliche Zusätze können enthalten sein Fettkörper, wie mineralische und synthetische Öle, wie z.B. Paraffine, Siliconöle und aliphatische Kohlenwasserstoffe mit mehr als 8 Kohlenstoffatomen, tierische und pflanzliche Öle, wie z.B. Sonnenblumenöl, Kokosöl, Avocadoöl, Olivenöl, Lanolin, oder Wachse, Fettsäuren, Fettsäureester, wie z.B. Triglyceride von $C_6$-$C_{30}$-Fettsäuren, Wachsester, wie z.B. Jojobaöl, Fettalkohole, Vaseline, hydriertes Lanolin und azetyliertes Lanolin. Selbstverständlich können auch Mischungen derselben verwendet werden.

Übliche Verdickungsmittel in derartigen Formulierungen sind vernetzte Polyacrylsäuren und deren Derivate, Polysaccharide wie Xanthan-Gum, Agar-Agar, Alginate oder Tylosen, Carboxymethylcellulose oder Hydroxycarboxymethylcellulose, Fettalkohole, Monoglyceride und Fettsäuren, Polyvinylakolhol und Polyvinylpyrrolidon.

Man kann die Polymere auch mit herkömmlichen Polymeren abmischen, falls spezielle Eigenschaften eingestellt werden sollen.

Als herkömmliche Polymere eignen sich beispielsweise anionische, kationische, amphotere und neutrale Polymere.

Beispiele für anionische Polymere sind Homo- und Copolymerisate von Acrylsäure und Methacrylsäure oder deren Salze, Copolymere von Acrylsäure und Acrylamid und deren Salze; Natriumsalze von Polyhydroxycarbonsäuren, wasserlösliche oder wasserdispergierbare Polyester, Polyurethane und Polyharnstoffe. Besonders geeignete Polymere sind Copolymere aus t-Butylacrylat, Ethylacrylat, Methacrylsäure (z.B. Luvimer® 100P), Copolymere aus Ethylacrylat und Methacrylsäure (z.B. Luvimer® MAE), Copolymere aus N-tert.-Butylacrylamid, Ethylacrylat, Acrylsäure (Ultrahold® 8, strong), Copolymere aus Vinylacetat, Crotonsäure und gegebenenfalls weitere Vinylester (z.B. Luviset® Marken), Maleinsäureanhydridcopolymere, ggf. mit Alkoholen umgesetzt, anionische Polysiloxane, z.B. carboxyfunktionelle, Copolymere aus Vinylpyrrolidon, t-Butylacrylat, Methacrylsäure (z.B Luviskol® VBM), Copolymere von Acrylsäure und Methacrylsäure mit hydrophoben Monomeren, wie z.B. $C_4$-$C_{30}$-Alkylester der Meth(acrylsäure), $C_4$-$C_{30}$-Alkylvinylester, $C_4$-$C_{30}$-Alkylvinylether und Hyaluronsäure.

Weitere geeignete Polymere sind kationische Polymere mit der Bezeichnung Polyquaternium nach INCI, z.B. Copolymere aus Vinylpyrrolidon/N-Vinylimidazoliumsalzen (Luviquat® FC, Luviquat® HM, Luviquat® MS, Luviquat® Care), Copolymere aus N-Vinylpyrrolidon/ Dimethylaminoethylmethacrylat, quaternisiert mit Diethylsulfat (Luviquat® PQ 11), Copolymere aus N-Vinylcaprolactam/N-Vinylpyrrolidon/N-Vinylimidazoliumsalzen (Luviquat® Hold); kationische Cellulosederivate (Polyquaternium-4 und -10), Acrylamidcopolymere (Polyquaternium-7) und Chitosan.

Als weitere Polymere sind auch neutrale Polymere geeignet wie Polyvinylpyrrolidone, Copolymere aus N-Vinylpyrrolidon und Vinylacetat und/oder Vinylpropionat, Polysiloxane, Polyvinylcaprolactam und Copolymere mit N-Vinylpyrrolidon, Polyethylenimine und deren Salze, Polyvinylamine und deren Salze, Cellulosederivate, Polyasparaginsäuresalze und Derivate.

Zur Einstellung bestimmter Eigenschaften können die Zubereitungen zusätzlich auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane oder Silikonharze.

Die Copolymerisate werden in kosmetischen Zubereitungen eingesetzt, deren Herstellung nach den üblichen dem Fachmann geläufigen Regeln erfolgt.

Solche Formulierungen liegen vorteilhafterweise in Form von Emulsionen bevorzugt als Wasser-in-Öl-(W/O)- oder Öl-in-Wasser-(O/W)-Emulsionen vor. Es ist aber auch erfindungsgemäß möglich und gegebenenfalls vorteilhaft andere Formulierungsarten zu wählen, beispielsweise Hydrodispersionen, Gele, Öle, Oleogele, multiple Emulsionen, beispielsweise in Form von W/O/W- oder O/W/O-Emulsionen, wasserfreie Salben bzw. Salbengrundlagen usw.

Die Herstellung brauchbarer Emulsionen erfolgt nach bekannten Methoden.

Die Emulsionen enthalten neben dem Copolymer übliche Bestandteile, wie Fettalkohole, Fettsäureester und insbesondere Fettsäuretriglyceride, Fettsäuren, Lanolin und Derivate davon, natürliche oder synthetische Öle oder Wachse und Emulgatoren in Anwesenheit von Wasser.

Die Auswahl der Emulsionstyp-spezifischen Zusätze und die Herstellung geeigneter Emulsionen ist beispielsweise beschrieben in Schrader, Grundlagen und Rezepturen der Kosmetika, Hüthig Buch Verlag, Heidelberg, 2. Auflage, 1989, Dritter Teil.

So kann eine brauchbare Hautcreme z.B. als W/O-Emulsion vorliegen. Eine derartige Emulsion enthält eine wässrige Phase, die mittels eines geeigneten Emulgatorsystems in einer Öl- oder Fettphase emulgiert ist.

Die Konzentration des Emulgatorsystems beträgt in diesem Emulsions-Typ etwa 4 und 35 Gew.-%, bezogen auf das Gesamtgewicht der Emulsion; die Fettphase macht etwa 20 und 60 Gew.-% aus und die wässrige Phasen etwa 20 und 70 Gew.-%, jeweils bezogen auf das Gesamtgewicht der Emulsion. Bei den Emulgatoren handelt es sich um diejenigen, welche in diesem Emulsionstyp üblicherweise verwendet werden. Sie werden z.B. ausgewählt unter: $C_{12}$-$C_{18}$-Sorbitan-Fettsäureestern; Estern von Hydroxystearinsäure und $C_{12}$-$C_{30}$-Fettalkoholen; Mono- und Diestern von $C_{12}$-$C_{18}$-Fettsäuren und Glyzerin oder Polyglyzerin; Kondensaten von Ethylenoxid und Propylenglycolen; oxypropylenierten/oxyethylenierten $C_{12}$-$C_{20}$-Fettalkoholen; polycyclischen Alkoholen, wie Sterolen; aliphatischen Alkoholen mit einem hohen Molekulargewicht, wie Lanolin; Mischungen von oxypropylenierten/polyglycerinierten Alkoholen und Magnesiumisostearat; Succinestern von polyoxyethylenierten oder polyoxypropylenierten Fettalkoholen; und Mischungen von Magnesium-, Calcium-, Lithium-, Zink- oder Aluminiumlanolat und hydriertem Lanolin oder Lanolin-alkohol.

Zu geeigneten Fettkomponenten, welche in der Fettphase der Emulsionen enthalten sein können, zählen Kohlenwasserstofföle, wie Paraffinöl, Purcellinöl, Perhydrosqualen und Lösungen mikrokristalliner Wachse in diesen Ölen; tierische oder pflanzliche Öle, wie Süßmandelöl, Avocadoöl, Calophylumöl, Lanolin und Derivate davon, Ricinusöl, Sesamöl, Olivenöl, Jojobaöl, Karité-Öl, Hoplostethus-Öl; mineralische Öle, deren Destillationsbeginn unter Atmosphärendruck bei ca. 250°C und deren Destillationsendpunkt bei 410°C liegt, wie z.B. Vaselinöl; Ester gesättigter oder ungesättigter Fettsäuren, wie Alkylmyristate, z.B. i-Propyl-, Butyl- oder Cetylmyristat, Hexadecylstearat, Ethyl- oder i-Propylpalmitat, Octan- oder Decansäuretriglyceride und Cetylricinoleat.

Die Fettphase kann auch in anderen Ölen lösliche Siliconöle, wie Dimethylpolysiloxan, Methylphenylpolysiloxan und das Silikonglycol-Copolymer, Fettsäuren und Fettalkohole enthalten.

Um die Retention von Ölen zu begünstigen, kann man auch Wachse verwenden, wie z.B. Carnauba-Wachs, Candellilawachs, Bienenwachs, mikrokristallines Wachs, Ozokeritwachs und Ca-, Mg- und Al-Oleate, -Myristate, -Linoleate und -Stearate.

Im allgemeinen werden diese Wasser-in-Öl-Emulsionen so hergestellt, dass die Fettphase und der Emulgator in den Ansatzbehälter gegeben werden. Man erwärmt diesen bei einer Temperatur von 70 bis 75°C, gibt dann die in Öl löslichen Ingredienzen zu und fügt unter Rühren Wasser hinzu, welches vorher auf die gleiche Temperatur erwärmt wurde und worin man die wasserlöslichen Ingredienzen vorher gelöst hat; man rührt, bis man eine Emulsion der gewünschten Feinheit hat, lässt sie dann auf Raumtemperatur abkühlen, wobei gegebenenfalls weniger gerührt wird.

Weiterhin kann eine Pflegeemulsion als O/W-Emulsion vorliegen. Eine derartige Emulsion enthält üblicherweise

eine Ölphase, Emulgatoren, die die Ölphase in der Wasserphase stabilisieren, und eine wässrige Phase, die üblicherweise verdickt vorliegt.

Die wässrige Phase der O/W-Emulsion der Zubereitungen enthält gegebenenfalls

- Alkohole, Diole oder Polyole sowie deren Ether, vorzugsweise Ethanol, Isopropanol, Propylenglycol, Glycerin, Ethylenglycolmonoethylether;
- übliche Verdickungsmittel bzw. Gelbildner, wie z.B. vernetzte Polyacrylsäuren und deren Derivate, Polysaccharide wie Xanthan Gum oder Alginate, Carboxymethylcellulose oder Hydroxycarboxymethylcellulose, Fettalkohole, Polyvinylalkohol und Polyvinylpyrrolidon.

Die Ölphase enthält in der Kosmetik übliche Ölkomponenten, wie beispielsweise:

- Ester aus gesättigten und/oder ungesättigten, verzweigten und/oder unverzweigten $C_3$-$C_{30}$-Alkancarbonsäuren und gesättigten und/oder ungesättigten, verzweigten und/oder unverzweigten $C_3$-$C_{30}$-Alkoholen, aus aromatischen Carbonsäuren und gesättigten und/oder ungesättigten, verzweigten und/oder unverzweigten $C_3$-$C_{30}$-Alkoholen, beispielhaft Isopropylmyristat, Isopropylstearat, Hexyldecylstearat, Oleyloleat; außerdem synthetische, halbsynthetische und natürliche Gemische solcher Ester, wie Jojobaöl;
- verzweigte und/oder unverzweigte Kohlenwasserstoffe und -wachse;
- Silikonöle wie Cyclomethicon, Dimethylpolysiloxan, Diethylpolysiloxan, Octamethylcyclotetrasiloxan sowie Mischungen daraus;
- Dialkylether;
- Mineralöle und Mineralwachse;
- Triglyceride gesättigter und/oder ungesättigter, verzweigter und/oder unverzweigter $C_8$-$C_{24}$-Alkancarbonsäuren; sie können ausgewählt werden aus synthetischen, halbsynthetischen oder natürlichen Ölen, wie Olivenöl, Palmöl, Mandelöl oder Mischungen.

Als Emulgatoren kommen vorzugsweise O/W-Emulgatoren, wie Polyglycerinester, Sorbitanester oder teilveresterte Glyceride, in Betracht.

Die Herstellung kann durch Aufschmelzen der Ölphase bei ca. 80°C erfolgen; die wasserlöslichen Bestandteile werden in heißem Wasser gelöst, langsam und unter Rühren zur Ölphase zugegeben; homogenisiert und kaltgerührt.

Die Polymere eignen sich auch zur Verwendung in Wasch- und Duschgel-Formulierungen sowie Badepräparaten.

Solche Formulierungen enthalten neben den erfindungsgemäßen Polymeren üblicherweise anionische Tenside als Basistenside und amphotere und nichtionische Tenside als Cotenside, sowie Lipide, Parfümöle, Farbstoffe, organische Säuren, Konservierungsstoffe und Antioxidantien sowie Verdicker/Gelbildner, Hautkonditioniermittel und Feuchthaltemittel.

In den Wasch, Dusch- und Badepräparaten können alle in Körperreinigungsmitteln üblicherweise eingesetzte anionische, neutrale, amphotere oder kationische Tenside verwendet werden.

Die Formulierungen enthalten 2 bis 50 Gew.-% Tenside, bevorzugt 5 bis 40 Gew.-%, besonders bevorzugt 8 bis 30 Gew-%.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxideinheiten im Molekül aufweisen.

Geeignet sind zum Beispiel Natriumlaurylsulfat, Ammoniumlaurylsulfat, Natriumlaurylethersulfat, Ammoniumlaurylethersulfat, Natriumlaurylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind zum Beispiel Alkylbetaine, Alkylamidopropylbetaine, Alkylsulfobetaine, Alkylglycinate, Alkylcarboxyglycinate, Alkylamphoacetate- oder -propionate, Alkylamphodiacetate, oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono- oder Dialkylalkanolamide, Fettsäure-ester von Polyethylenglykolen, ethoxylierte Fettsäureamide, Alkylpolyglykoside oder Sorbitanetherester geeignet.

Außerdem können die Wasch, Dusch- und Badepräparate übliche kationische Tenside enthalten, wie z.B. quaternäre Ammoniumverbindungen, beispielsweise Cetyltrimethylammoniumchlorid.

Zusätzlich können auch weitere übliche kationische Polymere eingesetzt werden, so z.B. Copolymere aus Acrylamid und Dimethyldiallylammoniumchlorid (Polyquaternium-7), kationische Cellulosederivate (Polyquaternium-4, -10), Guar-hydroxypropyltrimethylammoniumchlorid (INCI: Hydroxypropyl Guar Hydroxypropyltrimonium Chloride), Copolymere aus N-Vinylpyrrolidon und quaternisiertem N-Vinylimidazol (Polyquaternium-16, -44, -46), Copolymere aus N-Vinypyrrolidon/Dimethylaminoethyl-methacrylat, quaternisiert mit Diethylsulfat (Polyquaternium-11) und andere.

Weiterhin können die Wasch- und Duschgel-Formulierungen und Badepräparate Verdicker, wie z.B. Kochsalz, PEG-55, Propylene Glycol Oleate, PEG-120 Methyl Glucose Dioleate und andere, sowie Konservierungsmittel, weitere Wirk- und Hilfsstoffe und Wasser enthalten.

Haarkosmetische Zubereitungen umfassen insbesondere Stylingmittel und/oder Konditioniermittel in haarkosmetischen Zubereitungen wie Haarkuren, Haarschäume (engl. Mousses), (Haar)gelen oder Haarsprays, Haarlotionen, Haarspülungen, Haarshampoos, Haaremulsionen, Spitzenfluids, Egalisierungsmittel für Dauerwellen, Haarfärbe- und -bleichmittel, "Hot-Oil-Treatment"-Präparate, Conditioner, Festigerlotionen oder Haarsprays. Je nach Anwendungsgebiet können die haarkosmetischen Zubereitungen als (Aerosol-)Spray, (Aerosol-)Schaum, Gel, Gelspray, Creme, Lotion oder Wachs appliziert werden.

Die haarkosmetischen Formulierungen der erfindungsgemäßen Verwendung enthalten in einer bevorzugten Ausführungsform

a) 0,05 bis 20 Gew.-% des Polymers

b) 20 bis 99,95 Gew.-% Wasser und/oder Alkohol

c) 0 bis 79,5 Gew.-% weitere Bestandteile

Unter Alkohol sind alle in der Kosmetik üblichen Alkohole zu verstehen, z.B. Ethanol, Isopropanol, n-Propanol.

Unter weiteren Bestandteilen sind die in der Kosmetik üblichen Zusätze zu verstehen, beispielsweise Treibmittel, Entschäumer, grenzflächenaktive Verbindungen, d.h. Tenside, Emulgatoren, Schaumbildner und Solubilisatoren. Die eingesetzten grenzflächenaktiven Verbindungen können anionisch, kationisch, amphoter oder neutral sein. Weitere übliche Bestandteile können ferner sein z.B. Konservierungsmittel, Parfümöle, Trübungsmittel, Wirkstoffe, UV-Filter, Pflegestoffe wie Panthenol, Collagen, Vitamine, Eiweißhydrolysate, Alpha- und Beta-Hydroxycarbonsäuren, Eiweißhydrolysate, Stabilisatoren, pH-Wert-Regulatoren, Farbstoffe, Viskositätsregulierer, Gelbildner, Farbstoffe, Salze, Feuchthaltemittel, Rückfetter, Komplexbildner und weitere übliche Additive.

Weiterhin zählen hierzu alle in der Kosmetik bekannten Styling- und Conditionerpolymere, die in Kombination mit den Polymerisaten eingesetzt werden können, falls ganz spezielle Eigenschaften eingestellt werden sollen.

Als herkömmliche Haarkosmetik-Polymere eignen sich beispielsweise anionische Polymere. Solche anionischen Polymere sind Homo- und Copolymerisate von Acrylsäure und Methacrylsäure oder deren Salze, Copolymere von Acrylsäure und Acrylamid und deren Salze; Natriumsalze von Polyhydroxycarbonsäuren, wasserlösliche oder wasserdispergierbare Polyester, Polyurethane (Luviset® P.U.R.) und Polyharnstoffe. Besonders geeignete Polymere sind Copolymere aus t-Butylacrylat, Ethylacrylat, Methacrylsäure (z.B. Luvimer® 100P), Copolymere aus N-tert.-Butylacrylamid, Ethylacrylat, Acrylsäure (Ultrahold® 8, Strong), Copolymere aus Vinylacetat, Crotonsäure und gegebenenfalls weiteren Vinylestern (z.B. Luviset® Marken), Maleinsäureanhydridcopolymere, ggf. mit Alkoholen umgesetzt, anionische Polysiloxane, z.B. carboxyfunktionelle, Copolymere aus Vinylpyrrolidon, t-Butylacrylat, Methacrylsäure (z.B Luviskol® VBM).

Weiterhin umfasst die Gruppe der zur Kombination mit den Polymerisaten geeigneten Polymere beispielhaft Balancer CR (National Starch; Acrylatcopolymer), Balancer 0/55 (National Starch; Acrylatcopolymer), Balancer 47 (National Starch; Octylacrylamid/Acrylat/Butylaminoethylmethacrylate-Copolymer), Aquaflex® FX 64 (ISP; Isobutylen/Ethylmaleimid/Hydroxyethylmaleimid-Copolymer), Aquaflex® SF-40 (ISP / National Starch; VP/Vinyl Caprolactam/DMAPA Acrylatcopolymer), Allianz® LT-120 (ISP / Rohm & Haas; Acrylat/C1-2 Succinat/Hydroxyacrylat-Copolymer), Aquarez® HS (Eastman; Polyester-1), Diaformer® Z-400 (Clariant; Methacryloylethylbetain/Methacrylat-Copolymer), Diaformer® Z-711 (Clariant; Methacryloylethyl N-oxid/Methacrylat-Copolymer), Diaformer® Z-712 (Clariant; Methacryloylethyl N-oxide/Methacrylat-Copolymer), Omnirez® 2000 (ISP; Monoethylester von Poly(Methylvinylether/Maleinsäure in Ethanol), Amphomer® HC (National Starch; Acrylat/ Octylacrylamid-Copolymer), Amphomer® 28-4910 (National Starch; Octyl-acrylamid/ Acrylat/Butylaminoethylmethacrylat-Copolymer), Advantage® HC 37 (ISP; Terpolymer aus Vinylcaprolactam/Vinylpyrrolidon/Dimethylaminoethylmethacrylat), Acudyner 258 (Rohm & Haas; Acrylat/ Hydroxyesteracrylat-Copolymer), Luviset® PUR (BASF, Polyurethane-1), Luviflex® Silk (BASF), Eastman® AQ48 (Eastman).

Ganz besonders bevorzugt werden als anionische Polymere Acrylate mit einer Säurezahl größer gleich 120 und Copolymere aus t-Butylacrylat, Ethylacrylat, Methacrylsäure.

Weitere geeignete Haarkosmetik-Polymere sind kationische Polymere mit der Bezeichnung Polyquaternium nach INCI, z.B. Copolymere aus Vinylpyrrolidon/N-Vinylimidazoliumsalzen (Luviquat® FC, Luviquat® HM, Luviquat® MS, Luviquat® Care), Copolymere aus N-Vinylpyrrolidon/Dimethylaminoethylmethacrylat, quaternisiert mit Diethylsulfat (Luviqxiat® PQ 11), Copolymere aus N-Vinylcaprolactam N-Vinylpyrrolidon/N-Vinylimidazoliumsalzen (Luviquat® Hold); kationische Cellulosederivate (Polyquaternium-4 und -10), Acrylamidcopolymere (Polyquaternium-7).

Ferner können kationische Guarderivate wie Guarhydroxypropyltrimoniumchlorid (INCI) verwendet werden.

Als weitere Haarkosmetik-Polymere sind auch neutrale Polymere geeignet wie Polyvinylpyrrolidone, Copolymere aus N-Vinylpyrrolidon und Vinylacetat und/oder Vinylpropionat, Polysiloxane, Polyvinylcaprolactam und Copolymere mit N-Vinylpyrrolidon, Polyethylenimine und deren Salze, Polyvinylamine und deren Salze, Cellulosederivate, Polyasparaginsäuresalze und Derivate.

Zur Einstellung bestimmter Eigenschaften können die Zubereitungen zusätzlich auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane, Silikonharze oder Dimethicon Copolyole (CTFA) und aminofunktionelle Silikonverbindungen wie Amodimethicone (CTFA).

Die Polymerisate eignen sich insbesondere als Festigungsmittel in Haarstyling-Zubereitungen, insbesondere Haarsprays (Aerosolsprays und Pumpsprays ohne Treibgas) und Haarschäume (Aerosolschäume und Pumpschäume ohne Treibgas).

In einer bevorzugten Ausführungsform enthalten diese Zubereitungen

a) 0,1 bis 10 Gew.-% des Polymers
b) 20 bis 99,9 Gew.-% Wasser und/oder Alkohol
c) 0 bis 70 Gew.-% eines Treibmittel
d) 0 bis 20 Gew.-% weitere Bestandteile

Treibmittel sind die für Haarsprays oder Aerosolschäume üblich verwendeten Treibmittel. Bevorzugt sind Gemische aus Propan/ Butan, Pentan, Dimethylether, 1,1-Difluorethan (HFC-152 a), Kohlendioxid, Stickstoff oder Druckluft.

Eine bevorzugte Formulierung für Aerosolhaarschäume enthält

a) 0,1 bis 10 Gew.-% des Polymers
b) 55 bis 99,8 Gew.-% Wasser und/oder Alkohol
c) 5 bis 20 Gew.-% eines Treibmittel
d) 0,1 bis 5 Gew.-% eines Emulgators
e) 0 bis 10 Gew.-% weitere Bestandteile

Als Emulgatoren können alle in Haarschäumen üblicherweise eingesetzten Emulgatoren verwendet werden. Geeignete Emulgatoren können nichtionisch, kationisch bzw. anionisch oder amphoter sein.

Beispiele für nichtionische Emulgatoren (INCI-Nomenklatur) sind Laurethe, z.B. Laureth-4; Cetethe, z.B. Cetheth-1, Polyethylenglycolcetylether; Ceteareth, z.B. Ceteareth-25, Polyglycolfettsäureglyceride, hydroxyliertes Lecithin, Lactylester von Fettsäuren, Alkylpolyglycoside.

Beispiele für kationische Emulgatoren sind Cetyldimethyl-2-hydroxyethylammoniumdihydrogenphosphat, Cetyltrimoniumchlorid, Cetyltrimmoniumbromid, Cocotrimoniummethylsulfat, Quaternium-1 bis x (INCI).

Anionische Emulgatoren können beispielsweise ausgewählt werden aus der Gruppe der Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Eine für Styling-Gele geeignete Zubereitung kann beispielsweise wie folgt zusammengesetzt sein:

a) 0,1 bis 10 Gew.-% des Polymerisates
b) 60 bis 99,85 Gew.-% Wasser und/oder Alkohol
c) 0,05 bis 10 Gew.-% eines Gelbildners

d) 0 bis 20 Gew.-% weitere Bestandteile

Als Gelbildner können alle in der Kosmetik üblichen Gelbildner eingesetzt werden. Hierzu zählen leicht vernetzte Polyacrylsäure, beispielsweise Carbomer (INCI), Cellulosederivate, z.B. Hydroxypropylcellulose, Hydroxyethylcellulose, kationisch modifizierte Cellulosen, Polysaccharide, z.B. Xanthum Gummi, Caprylic/Capric Triglyceride, Sodium acrylates Copolymer, Polyquaternium-32 (and) Paraffinum Liquidum (INCI), Sodium Acrylates Copolymer (and) Paraffinum Liquidum (and) PPG-1 Trideceth-6, Acrylamidopropyl Trimonium Chloride/Acrylamide Copolymer, Steareth-10 Allyl Ether Acrylates Copolymer, Polyquaternium-37 (and) Paraffinum Liquidum (and) PPG-1 Trideceth-6, Polyquaternium 37 (and) Propylene Glycole Dicaprate Dicaprylate (and) PPG-1 Trideceth-6, Polyquaternium-7, Polyquaternium-44.

Die Polymere können auch in Shampooformulierungen als Festigungs- und/oder Konditioniermittel eingesetzt werden. Als Konditioniermittel eignen sich insbesondere Polymere mit kationischer Ladung. Bevorzugte Shampooformulierungen enthalten

a) 0,05 bis 10 Gew.-% des Polymers
b) 25 bis 94,95 Gew.-% Wasser
c) 5 - 50 Gew.-% Tenside
c) 0 - 5 Gew.-% eines weiteren Konditioniermittels
d) 0 - 10 Gew.-% weitere kosmetische Bestandteile

In den Shampooformulierungen können alle in Shampoos üblicherweise eingesetzte anionische, neutrale, amphotere oder kationische Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Geeignet sind zum Beispiel Natriumlaurylsulfat, Ammoniumlaurylsulfat, Natriumlaurylethersulfat, Ammoniumlaurylethersulfat, Natriumlauroylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind zum Beispiel Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono- oder Dialkylalkanolamide, Fettsäureester von Polyethylenglykolen, Alkylpolyglykoside oder Sorbitanetherester geeignet.

Außerdem können die Shampooformulierungen übliche kationische Tenside enthalten, wie z.B. quaternäre Ammoniumverbindungen, beispielsweise Cetyltrimethylammoniumchlorid.

In den Shampooformulierungen können zur Erzielung bestimmter Effekte übliche Konditioniermittel in Kombination mit den erfindungsgemäßen Polymerisaten eingesetzt werden. Hierzu zählen beispielsweise kationische Polymere mit der Bezeichnung Polyquaternium nach INCI, insbesondere Copolymere aus Vinylpyrrolidon/N-Vinylimidazoliumsalzen (Luviquat® FC, Luviquat® HM, Luviquat® MS, Luviquat® Care), Copolymere aus N-Vinylpyrrolidon/Dimethylaminoethylmethacrylat, quaternisiert mit Diethylsulfat (Luviquat® PQ 11), Copolymere aus N-Vinylcaprolactam/N-Vinylpyrrolidon/N-Vinylimidazoliumsalzen (Luviquat® Hold); kationische Cellulosederivate (Polyquaternium-4 und -10), Acrylamidcopolymere (Polyquaternium-7). Ferner können Eiweißhydrolysate verwendet werden, sowie konditionierende Substanzen auf Basis von Silikonverbindungen, beispielsweise Polyalkylsiloxane, Polyarylsiloxane, Polyarylalkylsiloxane, Polyethersiloxane oder Silikonharze. Weitere geeignete Silikonverbindungen sind Dimethicon Copolyole (CTFA) und aminofunktionelle Silikonverbindungen wie Amodimethicone (CTFA). Ferner können kationische Guarderivate wie Guarhydroxypropyltrimoniumchlorid (INCI) verwendet werden.

Tabelle 2

| Eintrag | X TAA | Y MCOH | Y $PE(MC)_4$ | k-Wert 5 % NaCl Lösung | Feststoff gehalt | Tel Quel Viskosität | Kämmkraftreduktion Nass | | Trocken | |
|---|---|---|---|---|---|---|---|---|---|---|
| | Monomer (d) | Monomer (e) | Monomer (e) | | (%) | (mPas) | (note) | (%) | (note) | (%) |
| 1 | 0,75 | 0,3 | - | 73,4 | 10 | 450 | 1-2 | 53% | 1-2 | 72% |
| 2 | 0,75 | 0,3 | - | 77,8 | 12 | 12800 | 1-2 | 51% | 1-2 | 80% |
| 3 | 0,75 | - | 0,3 | 88,7 | 10 | 3700 | 1-2 | 49% | 1-2 | 83% |
| V1 | 0,75 | - | - | n.m. | 12 | >50000 | 1-2 | 52% | 1-2 | 74% |
| V2 | - | 0,3 | - | 24 | 13 | 150 | 2- | 13% | 2+ | 34% |
| V3 | 0,75 | - | - | 83 | 7 | 3200 | 1-2 | 50% | 1-2 | 75% |

Verwendete Abkürzungen (Gew.-% jeweils bezogen auf Gesamtmonomermenge)
TAA = Triallylamin (Gew.-%)
MCOH = Mercaptoethanol (Gew.-%)
$PE(MC)_4$ = Pentaerythrit tetramercaptoacetat (Gew.-%)
n.m. = Nicht Messbar durch zu große Menge Gelpartikeln in der Lösung

Die Viskosität wurde bestimmt mit einem Brookfield Viskosimeter mit Spindel 4, 12 Upm bei 25°C.

Beispiele

A: Herstellung der Polymere 1 bis 3

In einer Rührapparatur wurden 85,9 g N-Vinylpyrrolidon, 47,7 g 3-Methyl-1-vinylimidazoliuromethylsulfat (45 % in Wasser), X g Triallylamin (Menge in Tabelle 1) Y g Regler (Menge und Regler in Tabelle 1) und unterschiedliche Mengen Wasser (750 bis 920 g abhängig von Feststoffgehalt; Angabe zur Feststoffgehalt in Tabelle 1), vorgelegt und unter Rühren im Stickstoffstrom auf 65°C aufgeheizt. Dann wurde 25 g eines Zulaufs, bestehend aus 1,6 g 2,2'-Azo-bis(2-amidinopropan)dihydrochlorid in 65 g Wasser, in 3 Stunden zudosiert. Nach Ende des Zulaufs ließ man eine weitere Stunde bei 65°C rühren. Danach wurde die Polymerisationstemperatur erhöht bis 70°C und wurde der restliche Zulauf des 2,2'-Azo-bis(2-amidinopropan)dihydrochlorids über 1 weitere Stunden zugegeben. Nach Ende dieser Zulauf wurde für 2 weitere Stunden gerührt.
Man erhielt farblose Polymerlösungen mit unterschiedlichen Feststoffgehalten und Viskosität (Angaben in Tabelle 2).
Die Vergleichsversuche wurden entsprechend durchgeführt mit den in Tabelle 1 genannten Komponenten.

B: Vergleich der Eigenschaften der Polymerisate mit den Polymerisaten der Vergleichsbeispiele

Zur anwendungstechnischen Untersuchung wurden die Polymere in einer Tensidlösung-Rezeptur mit nachfolgender Zusammensetzung eingesetzt:

40,0 % Texapon NSO (Sodium Laureth Sulfat Lösung 28 %; Cognis)
10,0 % Tego-Betain L7 (Cocamidopropyl Betain Lösung 30 %; Goldschmidt)
0,5 % Polymerisat (Feststoffgehalt) ad 100 % Wasser Bestimmung der Kämmbarkeit

Die folgende Arbeitsanleitung beschreibt die Vorgehensweise zur Bestimmung der Nass- und Trockenkämmbarkeit von Haaren nach der Behandlung mit Konditioniermitteln. Alle Messungen wurden im Klimaraum bei 65 % relativer Feuchte und 21°C durchgeführt.

Verwendete Geräte

| | |
|---|---|
| Nasskämmbarkeit: | Frank Zug/Druck-Prüfgerät |
| Trockenkämmbarkeit: | Diastron Kraftmesssystem |
| Digitalwaage: | (Oberschalenwaage) |

Haare:

a) europäisch, gebleicht: Haartressen der Fa. Wernesgrün (Bleichung siehe unten)

b) asiatisch, unbehandelt: Haartressen der Fa. Wernesgrün mit gesplissten Spitzen

Folgende Prüfungen wurden durchgeführt:

- Nasskämmbarkeit nach Shampooanwendung an europäischen, gebleichten Haaren

- Trockenkämmbarkeit nach Shampooanwendung an asiatischen Haaren

Vorbehandlung / Reinigung der Haare:

Vor der Erstbenutzung wurden die asiatischen Haartressen in einem Lösungsmittelgemisch (Ethanol/Isopropanol/Aceton/Wasser 1:1:1:1) gereinigt bis die Haare im trockenen Zustand sauber (d.h. nicht mehr verklebt) aussehen. Anschließend wurden die Haare mit Natriumlaurylethersulfat gewaschen.

Die europäischen Haare wurden danach mit einer Bleichpaste (7,00 g Ammoniumcarbonat, 8,00 g Calciuncarbonat, 0,50 g Aerosil 200, 9,80 g Wasserstoffperoxid (30 %ig), 9,80 g Vollentsalztes Wasser) behandelt. Die Haartressen wurden in die Bleichpaste vollständig eingetaucht, so dass eine umfangreiche Benetzung der gesamten Haarooberfläche gewährleistet ist. Anschließend wurden die Tressen zwischen den Fingern abgestreift um die überschüssige Bleichpaste zu entfernen. Die Einwirkzeit, des somit verbleibenden Bleichmittels auf dem Haar, wird dem Grad der benötigten Schädigung angepasst, beträgt in der Regel 15 bis 30 Minuten, kann aber bedingt durch die Haarqualität schwanken. Danach wurden die gebleichten Haartressen unter fließendem Leitungswasser gründlich (2 Minuten) gespült und mit Natriumlaurylethersulfat gewaschen. Anschließend wurden die Haare wegen der sogenannten schleichenden Bleiche kurz in einer wässrigen, sauren Lösung (z.B. Citronensäure) eingetaucht und mit Leitungswasser nachgespült werden.

Anwendungen:

Die Haartresse wird 1 Minute in die zu testende Tensidformulierung getaucht, 1 Minute shampooniert und anschließend 1 Minute unter fließendem Trinkwasser (handwarm) ausgespült.

I) Nasskämmbarkeit

Bestimmung Blindwert Nasskämmbarkeit: Die gewaschenen Haare wurden über Nacht im Klimaraum getrocknet. Vor der Messung wurden sie zweimal mit Texapon NSO insgesamt 1 Minute shampooniert und 1 Minute ausgespült, damit sie definiert nass, d.h. gequollen sind. Vor Beginn der Messung wurde die Tresse so vorgekämmt, bis keine Verhakungen der Haare mehr vorhanden sind und somit bei wiederholtem Messkämmen eine konstante Kraftaufwendung erforderlich ist. Anschließend wurde die Tresse an der Halterung fixiert und mit der feinzinkigen Seite des Kammes in die feinzinkige Seite des Prüfkammes eingekämmt. Das Einlegen der Haare in den Prüfkamm erfolgte bei jeder Messung gleichmäßig und spannungsfrei. Die Messung wurde gestartet und mittels Software (EGRANUDO-Programm, Fa. Frank) ausgewertet. Die Einzelmessung wurde 5 bis 10 mal wiederholt. Der errechnete Mittelwert wurde notiert.

Bestimmung Messwert Nasskämmbarkeit: Nach der Bestimmung des Blindwertes wurden die Haare je nach gewünschter Anwendung behandelt. Die Messung der Kämmkraft erfolgt analog der Blindwertbestimmung.

Auswertung:

$$\text{Kämmkraftabnahme nass } [\%] = 100 - (\text{Messwert} * 100/\text{Blindwert})$$

II) Trockenkämmbarkeit

Bestimmung Blindwert Trockenkämmbarkeit: Die gewaschenen Haare wurden über Nacht im Klimaraum getrocknet. Vor Beginn der Messung wurde die Tresse so vorgekämmt, bis keine Verhakungen der Haare mehr vorhanden sind und somit bei wiederholtem Messkämmen eine konstante Kraftaufwendung erforderlich ist. Anschließend wurde die Tresse an der Halterung fixiert und in die feinzinkige Seite des Prüfkamms eingekämmt. Das Einlegen der Haare in den Prüfkamm erfolgte bei jeder Messung gleichmäßig und spannungsfrei. Die Messung wurde gestartet und mittels Software (mtt-win, Fa. DIASTRON) ausgewertet. Die Einzelmessung wurde 5- bis 10 mal wiederholt. Der errechnete Mittelwert wurde zusammen mit der Standardabweichung notiert.

Bestimmung Messwert Trockenkämmbarkeit: Nach der Bestimmung des Blindwertes wurden die Haare je nach gewünschter Anwendung behandelt und über Nacht getrocknet. Die Messung der Kämmkraft erfolgte analog der Blindwertbestimmung. Auswertung:

$$\text{Kämmkraftabnahme nass } [\%] = 100 - (\text{Messwert}*100/\text{Blindwert})$$

Die Ergebnisse sind in Tabelle 2 zusammengefasst.

Die erfindungsgemäß zu verwendenden Polymere (Beispiele 1 bis 3) zeigen gute anwendungstechnische Eigenschaften und lassen sich mit hohen Feststoffgehalten herstellen.

Im Vergleich dazu liefern Polymere, die ohne Monomer (e) hergestellt wurden, zu hohe Viskositäten (V-1) oder zu niedrigen Feststoffgehalt (V-3). Polymere, die durch Polymerisation in Anwesenheit eines Reglers aber ohne Vernetzer hergestellt wurden (V-2) zeigen gute Viskositäten, sind aber hinsichtlich ihrer anwendungstechnischen Eigenschaften unbefriedigend.

Die erfindungsgemäß zu verwendenden Polymere liefern, die Trockenkämmbarkeit, insbesondere aber die Nasskämmbarkeit betreffend, hervorragende Resultate. Ein weiterer Vorzug ist, dass mit dem Polymeren auch klare (Wasch-) Formulierungen möglich sind.

Ein weiterer Vorzug ist, dass die erfindungsgemäß zu verwendenden Polymerisate mit hohen Feststoffgehalten herstellbar sind.

Beispiel 11 - Klares Conditioner Shampoo

| % | Inhaltsstoff | INCI |
|---|---|---|
| 15,00 | Tego Betain L 7 | Cocamidopropylbetain |
| 10,00 | Amphotensid GB 2009 | Dinatrium Cocoamphodiacetat |
| 5,00 | Cremophor PS 20 | Polysorbat 20 |
| 5,00 | Plantacare 2000 | Decylglucosid |
| 3,00 | Stepan PEG 6000 DS | PEG-150 Distearat |
| q.s. | Parfum | |
| q.s. | Konservierungsmittel | |
| q.s. | Zitronensäure | |
| 0,1-1,0 | Aktive Substanz Conditionerpolymer gemäß Beispiel 1 | |
| 2,00 | Rewopal LA 3 | Laureth-3 |
| ad 100 | Wasser demineralisiert Aqua dem. | |

Beispiel 12 - Conditioner Shampoo

| % | Inhaltsstoff | INCI |
|---|---|---|
| 30,00 | Texapon NSO | Natrium Laurethsulfat |
| 6,00 | Dehyton G | Natrium Cocoamphoacetat |
| 6,00 | Dehyton K | Cocamidopropylbetain |
| 3,00 | Euperlan PK 771 | Natrium Laurethsulfate, Glycol Distearat, Cocamid MEA, Laureth-10 |
| 0,1-1,0 | Aktive Substanz Conditionerpolymer gemäß Beispiel 1 | |
| 2,00 | Dimethicone | |
| q.s. | Parfum | |
| q.s. | Konservierungsmittel | |
| q.s. | Zitronensäure | |
| 1,00 | Natriumchlorid | |
| ad 100 | Wasser demineralisiert Aqua dem. | |

Beispiel 13 - Conditioner Shampoo

| % | Inhaltsstoff | INCI |
|---|---|---|
| 30,00 | Texapon NSO | Natrium Laurethsulfat |
| 6,00 | Dehyton G | Natrium Cocoamphoacetat |
| 6,00 | Dehyton K | Cocamidopropylbetain |

(fortgesetzt)

| % | Inhaltsstoff | INCI |
|---|---|---|
| 3,00 | Euperlan PK 771 | Natrium Laurethsulfate, Glycol Distearat, Cocamid MEA, Laureth-10 |
| 0,1-1,0 | Aktive Substanz Conditionerpolymer gemäß Beispiel 2 | |
| 2,00 | Amidodimethicone | |
| q.s. | Parfum | |
| q.s. | Konservierungsmittel | |
| q.s. | Zitronensäure | |
| 1,00 | Natriumchlorid | |
| ad 100 | Wasser demineralisiert Aqua dem. | |

Beispiel 14 - Conditioner Shampoo

| % | Inhaltsstoff | INCI |
|---|---|---|
| 40,00 | Texapon NSO | Natrium Laurethsulfat |
| 10,00 | Dehyton K | Cocamidopropylbetain |
| 3,00 | Euperlan PK 771 | Natrium Laurethsulfat, Glycol Distearat, Cocamid MEA, Laureth-10 |
| 0,1-1,0 | Aktive Substanz Conditionerpolymer gemäß Beispiel 2 | |
| 2,00 | Dow Corning 3052 | |
| q.s. | Parfum | |
| q.s. | Konservierungsmittel | |
| q.s. | Zitronensäure | |
| 2,00 | Cocamido DEA | |
| ad 100 | Wasser demineralisiert Aqua dem. | |

Beispiel 15 - Conditioner Shampoo

| % | Inhaltsstoff | INCI |
|---|---|---|
| 30,00 | Texapon NSO | Natrium Laurethsulfate |
| 6,00 | Dehyton G | Sodium Cocoamphoacetat |
| 6,00 | Dehyton K | Cocamidopropylbetain |
| 3,00 | Euperlan PK 771 | Natrium Laurethsulfat, Glycol Distearat, Cocamid MEA, Laureth-10 |
| 0,1-1,0 | Aktive Substanz Conditionerpolymer gemäß Beispiel 2 | |
| 2,00 | Dimethicone | |
| q.s. | Parfum | |
| q.s. | Konservierungsmittel | |
| q.s. | Zitronensäure | |
| 2,00 | Cocamido DEA | |
| ad 100 | Wasser demineralisiert Aqua dem. | |

Beispiel 16 - Anti-Dandruff Shampoo

| % | Inhaltsstoff | INCI |
|---|---|---|
| 40,00 | Texapon NSO | Natrium Laurethsulfat |
| 10,00 | Tego Betain L 7 | Cocamidopropylbetain |
| 10,00 | Rewopol SB FA 30 | Dinatrium Laurethsulfosuccinat |

(fortgesetzt)

| % | Inhaltsstoff | INCI |
|---|---|---|
| 2,50 | Euperlan PK 771 | Natrium Laurethsulfat, Glycol Distearat, Cocamid MEA, Laureth-10 |
| 0,1-1,0 | Aktive Substanz Conditionerpolymer gemäß Beispiel 3 | |
| 0,50 | Crinipan AD | Climbazol |
| q.s. | Parfum | |
| q.s. | Konservierungsmittel | |
| q.s. | Zitronensäure | |
| 0,50 | Natrium Chlorid | |
| ad 100 | Wasser demineralisiert Aqua dem. | |

Beispiel 17 - Shampoo

| % | Inhaltsstoff | INCI |
|---|---|---|
| 25,00 | Sodium Laurethsulfat | |
| 5,00 | Cocamidopropylbetain | |
| 2,50 | Euperlan PK 771 | Natrium Laurethsulfat, Glycol Distearat, Cocamid MEA, Laureth-10 |
| 0,1-1,0 | Aktive Substanz Conditionerpolymer gemäß Beispiel 3 | |
| 2,0 | Cocamido DEA | |
| q.s. | Parfum | |
| q.s. | Konservierungsmittel | |
| ad 100 | Wasser demineralisiert Aqua dem. | |

Beispiel 18 - Shampoo

| % | Inhaltsstoff | INCI |
|---|---|---|
| 20,00 | Ammonium Laurethsulfat | |
| 15,00 | Ammonium Laurylsulfate | |
| 5,00 | Cocamidopropylbetain | |
| 2,50 | Euperlan PK 771 | Natrium Laurethsulfat, Glycol Distearat, Cocamid MEA, Laureth-10 |
| 0,1-1,0 | Aktive Substanz Conditionerpolymer gemäß Beispiel 3 | |
| q.s. | Parfum | |
| q.s. | Konservierungsmittel | |
| 0,50 | Natrium Chlorid | |
| ad 100 | Wasser demineralisiert Aqua dem. | |

Beispiel 19 - Shampoo

| % | Inhaltsstoff | INCI |
|---|---|---|
| 20,00 | Natrium Laurethsulfat | |
| 15,00 | Natrium Laurylsulfat | |
| 5,00 | Cocamidopropylbetain | |
| 2,50 | Euperlan PK 771 | Natrium Laurethsulfat, Glycol Distearat, Cocamid MEA, Laureth-10 |
| 0,1-1,0 | Aktive Substanz Conditionerpolymer gemäß Beispiel 2 | |
| q.s. | Parfum | |
| q.s. | Konservierungsmittel | |

(fortgesetzt)

| % | Inhaltsstoff | INCI |
|---|---|---|
| 0,50 | Natrium Chlorid | |
| ad 100 | Wasser demineralisiert | Aqua dem. |

Beispiel 20 - Klares Douche Gel

| % | Inhaltsstoff | INCI |
|---|---|---|
| 40,00 | Texapon NSO | Natrium Laurethsulfat |
| 5,00 | Plantacare 2000 | Decyl Glucosid |
| 5,00 | Tego Betain L 7 | Cocamidopropylbetain |
| 0,1-1,0 | Aktive Substanz Conditionerpolymer gemäß Beispiel 2 | |
| 1,00 | D-Panthenol USP | Panthenol |
| q.s. | Parfum | |
| q.s. | Konservierungsmittel | |
| q.s. | Zitronensäure | |
| 2,00 | Natrium Chlorid | |
| ad 100 | Wasser demineralisiert | Aqua dem. |

Beispiel 21 - Shampoo

| % | Inhaltsstoff | INCI |
|---|---|---|
| 12,00 | Texapon N 70 | Natrium Laurethsulfat |
| 1,50 | Plantacare 2000 | Decyl Glucosid |
| 2,50 | Dehyton PK 45 | Cocamidopropylbetain |
| 5,00 | Lamesoft PO 65 | Coco-Glucosid Glyceryloleat |
| 2,00 | Euperlan PK 771 | Natrium Laurethsulfate, Glycol Distearat, Cocamid MEA, Laureth-10 |
| 0,1-1,0 | Aktive Substanz Conditionerpolymer gemäß Beispiel 1 | |
| q.s. | Konservierungsmittel | |
| q.s. | Sicovit Sunset | Sunset Yellow C.I. 15 985 Yellow 85 E 110 |
| q.s. | Parfum | |
| 1,00 | Natrium Chlorid | |
| ad 100 | Wasser demineralisiert | |

Beispiel 22 - Shampoo

| % | Inhaltsstoff | INCI |
|---|---|---|
| 12,00 | Texapon N 70 | Natrium Laurethsulfat |
| 1,50 | Plantacare 2000 | Decyl Glucosid |
| 2,50 | Dehyton PK 45 | Cocamidopropylbetain |
| 5,00 | Lamesoft PO 65 | Coco-Glucosid Glyceryloleat |
| 2,00 | Euperlan PK 771 | Natrium Laurethsulfat, Glycol Distearat, Cocamid MEA, Laureth-10 |
| 0,1-1,0 | Aktive Substanz Conditionerpolymer gemäß Beispiel 1 | |
| q.s. | Konservierungsmittel | |
| q.s. | Sicovit Sunset | Sunset Yellow C.I. 15 985 Yellow 85 E 110 |
| q.s. | Parfum | |

(fortgesetzt)

| % | Inhaltsstoff | INCI |
|---|---|---|
| 1,00 | Natrium Chlorid | |
| ad 100 | Wasser demineralisiert | |

## Patentansprüche

1. Verwendung von Polymeren, die erhältlich sind durch radikalisch initiierte Copolymerisation von Monomergemischen aus

   (a) 2 bis 70 Gew.-% eines kationischen Monomeren ausgewählt aus der Gruppe bestehend aus 3-Methyl-1-vinyl-imidazoliumchlorid und -methosulfat,
   (b) 22 bis 97,98 Gew.-% N-Vinylpyrrolidon,
   (c) 0 bis 40 Gew.-% eines weiteren radikalisch copolymerisierbaren Monomeren,
   (d) 0,01 bis 8 Gew.-% mindestens einem als Vernetzer wirkenden Monomeren mit mindestens zwei ethylenisch ungesättigten, nichtkonjugierten Doppelbindungen und
   (e) 0,01 bis 8 Gew.-% eines Reglers in haarkosmetischen Zubereitungen.

2. Verwendung von Polymeren wie definiert in Anspruch 1 als Konditionierungsmittel in kosmetischen Zubereitungen.

3. Verwendung nach einem der Ansprüche 1 bis 2, wobei als Regler (e) Verbindungen verwendet werden, die Schwefel in gebundener Form enthalten.

4. Verwendung nach Anspruch 3, wobei als Regler Thiole verwendet werden.

## Claims

1. The use of polymers which are obtainable by free-radically initiated copolymerization of monomer mixtures of

   (a) 2 to 70% by weight of a cationic monomer selected from the group consisting of 3-methyl-1-vinylimidazolium chloride and 3-methyl-1-vinylimidazolium methosulfate,
   (b) 22 to 97.98% N-vinylpyrrolidone,
   (c) 0 to 40% by weight of a further free-radically copolymerizable monomer,
   (d) 0.01 to 8% by weight of at least one crosslinking monomer having at least two ethylenically unsaturated, non-conjugated double bonds and
   (e) 0.01 to 8% by weight of a regulator in hair cosmetic preparations.

2. The use of polymers as defined in claim 1 as conditioning agents in cosmetic preparations.

3. The use according to either of claims 1 and 2, wherein compounds which comprise sulfur in bonded form are used as regulator (e).

4. The use according to claim 3, wherein thiols are used as regulator.

## Revendications

1. Utilisation de polymères, qui peuvent être obtenus par copolymérisation initiée de manière radicalaire de mélanges de monomères composés de

   (a) 2 à 70 % en poids d'un monomère cationique choisi dans le groupe constitué par le chlorure de 3-méthyl-1-vinyl-imidazolium et le méthosulfate de 3-méthyl-1-vinyl-imidazolium,
   (b) 22 à 97,98 % en poids de N-vinylpyrrolidone,
   (c) 0 à 40 % en poids d'un autre monomère copolymérisable de manière radicalaire,
   (d) 0,01 à 8 % en poids d'au moins un monomère agissant en tant qu'agents de réticulation comportant au

moins deux doubles liaisons éthyléniquement insaturées, non conjuguées et
(e) 0,01 à 8 % en poids d'un régulateur

dans des préparations cosmétiques capillaires.

**2.** Utilisation de polymères tels que définis dans la revendication 1 en tant qu'agent de conditionnement dans des préparations cosmétiques.

**3.** Utilisation selon l'une quelconque des revendications 1 et 2, des composés qui contiennent du soufre sous forme liée étant utilisés en tant que régleur (e).

**4.** Utilisation selon la revendication 3, des thiols étant utilisés en tant que régleur.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0246580 A
- EP 0544158 A
- US 4859756 A
- EP 0715843 A
- DE 2821239 A
- US 4348380 A
- DE 3106974 A
- US 5275809 A
- EP 0522755 A
- EP 0521665 A
- EP 0521666 A
- US 4806345 A
- WO 9325595 A
- DE 3209224
- WO 9626229 A
- US 4058491 A
- DE 4213971 A
- EP 0624617 A
- EP 0027850 A
- WO 9637525 A
- WO 9735544 A
- DE 19731907 A
- EP 0893117 A
- EP 1064924 A


### In der Beschreibung aufgeführte Nicht-Patentliteratur


- **K.C. BERGER ; G. BRANDRUP.** Polymer Handbook. John Wiley & Sons, 1989, II/81-II/141
- **FIKENTSCHER.** *Cellulosechemie,* 1932, vol. 13, 58-64
- **SCHRADER.** Grundlagen und Rezepturen der Kosmetika. Hüthig Buch Verlag, 1989